# EUROPEAN PATENT SPECIFICATION

(11) **EP 4 351 485 B1**
(45) Date of publication and mention of the grant of the patent: **01.04.2026**
(21) Application number: 22856384.7
(22) Date of filing: 28.06.2022
(51) Int. Cl.: A61F 2/40

(54) **FENESTRATED BEARING GLENOID COMPONENT**
MIT FENSTERN VERSEHENE GLENOIDLAGERKOMPONENTE
COMPOSANT GLÉNOÏDE À PALIER FENÊTRÉ

(30) Priority: 12.08.2021 US 202163232249 P
(43) Date of publication of application: 17.04.2024
(73) Proprietor: Howmedica Osteonics Corp., Mahwah, NJ 07430 (US)
(72) Inventor: TERRILL, Lance, Nathan, Glounthaune, Co. Cork, T45 NC84 (IE); OLSON, Nicholas, Kenneth, Goshen, NY 10924 (US); NAYAK, Amith, Great Meadows, NJ 07838 (US); WOLFE, Alexander, Paul, Fort Wayne, IN 46804 (US)
(74) Representative: Zimmermann & Partner Patentanwälte mbB
(86) International application number: PCT/US2022/035217
(87) International publication number: WO 2023/018485

(56) References cited:
- FR-B1- 2 790 662
- US-A- 4 990 161
- US-A1- 2001 011 192
- US-A1- 2005 049 709
- US-A1- 2005 192 673
- US-A1- 2008 140 130
- US-A1- 2008 294 268
- US-A1- 2010 249 938
- US-A1- 2013 066 433
- US-A1- 2014 243 986
- US-A1- 2018 064 537
- US-A1- 2019 029 833
- US-B2- 7 608 109
- US-B2- 8 454 705

## Description

### FIELD OF DISCLOSURE

The present disclosure generally relates to glenoid implants for shoulder prosthesis.

### BACKGROUND

A shoulder prosthesis includes a glenoid implant intended to replace the glenoid cavity of the scapula and/or a humeral implant intended to replace the humeral head. The glenoid implant generally includes an articular body intended to articulate with the humeral head, and a fixation means to stabilize the articular body with respect to the scapula. Document FR 2 790 662 B1 describes a glenoid implant.

Currently, clinical literature shows a high rate of radiolucency around glenoid cemented, non-cemented, and hybrid components in long-term use of those glenoid implants. One issue is the potential for rocking of the implant in the superior-inferior direction. Currently, cemented implants provide good short- and mid-term fixation, but loosen over time. The current hybrid cemented-press fit implants show similar performance. Press fit implants show high loosening at mid- to long-term time points.

One of the challenges with press-fit glenoid implants is that it is difficult to reliably secure the implant to the bone, which is why hybrid cemented-press fit implants have increased in popularity. Adding a modular metal tray with screws is a solution that is used in many other joints, however in the shoulder there is often insufficient space for a modular tray. In addition, the modular metal tray is often more stiff than desired.

Thus, improved glenoid implant design that offers enhanced and durable primary fixation to the bone is desired.

### SUMMARY

Provided herein are various embodiments of a glenoid implant that is a monolithic polymer bearing incorporating a metallic insert that allows advancement of one or more bone screws through the bearing surface resulting in enhanced fixation with the glenoid. The glenoid implant is part of a shoulder joint implant system.

According to some embodiments, a glenoid implant is disclosed that comprises: an articular body including an articulation surface and a bone-facing surface on opposite side of the articular body; a metal tray attached to the bone-facing surface; and one or more holes provided in the articular body extending from the articulation surface through the articular body and the metal tray to accommodate a screw therethrough.

According to some embodiments, a glenoid implant is disclosed that comprises: an articular body comprising an articulation surface and a bone-facing surface on opposite side of the articular body; and one or more holes provided in the articular body extending from the articulation surface through the articular body to the bone-facing surface.

According to some embodiments, an implant system is disclosed that comprises: a first articular component that is a glenoid implant that comprises: an articular body comprising an articulation surface and a bone-facing surface on opposite side of the articular body; a metal tray attached to the bone-facing surface; and one or more holes provided in the articular body extending from the articulation surface through the articular body and the metal tray to accommodate a screw therethrough; and a second articular component that is a humeral head implant configured to engage the articulation surface of the glenoid implant.

According to some embodiments, an implant system is disclosed that comprises: a first articular component that is a glenoid implant that comprises: an articular body comprising an articulation surface and a bone-facing surface on opposite side of the articular body, wherein at least the articulation surface is formed of ultra-high-molecular-weight polyethylene (UHMWPE); and one or more holes provided in the articular body extending from the articulation surface through the articular body to accommodate a screw therethrough; and a second articular component that is a humeral head implant configured to engage the articulation surface of the glenoid implant, wherein the humeral head implant comprises an articulation surface made of virgin polyether ether ketone (PEEK).

### BRIEF DESCRIPTION OF THE DRAWINGS

The various embodiments of the inventive implant of the present disclosure will be described in more detail in conjunction with the following drawing figures. The structures in the drawing figures are illustrated schematically and are not necessarily intended to show actual dimensions or relative scale.
FIG. 1A is an illustration showing an isometric view of an example of the glenoid bearing implant of the present disclosure viewed from the articulating surface side.
FIG. 1B is an illustration showing an isometric view of the glenoid bearing implant of FIG. 1A viewed from the bone-contacting surface side.
FIG. 1C is an illustration showing a sectional view of the glenoid bearing implant of FIG. 1A.
FIG. 1D is an enlarged sectional view of a hole extending through the implant's articular body and a bone screw positioned therein according to the present disclosure.
FIG. 2A is an illustration showing an isometric view of another example of a glenoid bearing implant according to the present disclosure viewed from the articulating surface side.
FIG. 2B is an illustration showing an isometric view of the glenoid bearing implant of FIG. 2A viewed from the bone-contacting surface side.
FIG. 2C is an illustration of a sectional view of the glenoid bearing implant of FIGS. 2A and 2B.
FIGS. 2D and 2E are illustrations showing additional embodiments of the glenoid bearing implants.
FIGS. 3A-3E are illustrations showing another embodiment of the glenoid bearing implant according to the present disclosure.
FIG. 4 is a sectional view of a humeral head implant configured to engage the articular surface of the glenoid bearing implant of the present disclosure.

### DETAILED DESCRIPTION

This description of the exemplary embodiments is intended to be read in connection with the accompanying drawings, which are to be considered part of the entire written description. The drawing figures are not necessarily to scale and certain features may be shown exaggerated in scale or in somewhat schematic form in the interest of clarity and conciseness. In the description, relative terms such as "horizontal," "vertical," "up," "down," "top" and "bottom" as well as derivatives thereof (e.g., "horizontally," "downwardly," "upwardly," etc.) should be construed to refer to the orientation as then described or as shown in the drawing figure under discussion. These relative terms are for convenience of description and normally are not intended to require a particular orientation. Terms including "inwardly" versus "outwardly," "longitudinal" versus "lateral" and the like are to be interpreted relative to one another or relative to an axis of elongation, or an axis or center of rotation, as appropriate. Terms concerning attachments, coupling and the like, such as "connected" and "interconnected," refer to a relationship wherein structures are secured or attached to one another either directly or indirectly through intervening structures, as well as both movable or rigid attachments or relationships, unless expressly described otherwise. When only a single machine is illustrated, the term "machine" shall also be taken to include any collection of machines that individually or jointly execute a set (or multiple sets) of instructions to perform any one or more of the methodologies discussed herein. The term "operatively connected" is such an attachment, coupling or connection that allows the pertinent structures to operate as intended by virtue of that relationship. In the claims, means-plus-function clauses, if used, are intended to cover the structures described, suggested, or rendered obvious by the written description or drawings for performing the recited function, including not only structural equivalents but also equivalent structures.

Provided herein are various improved glenoid bearing implants that have articulation surface that is configured to engage with an anatomical humeral head or a humeral component of a shoulder replacement implant system. Therefore, references to "a humeral head" as used herein should be construed to include both an anatomical humeral head as well as implant humeral head.

The improved glenoid bearing implant is a monolithic polymer bearing that can, in some embodiments, include a metallic insert. The glenoid bearing implant has one or more holes extending through the implant body that allows advancement of one or more bone screws through the implant body to secure the glenoid bearing implant to a glenoid. This design retains the low stiffness of a polymer bearing with the high initial fixation power of screws.

In preferred embodiments, the opposing humeral head is also a polymer so that stresses in the polymer bearing are reduced. The screws used are preferably locking type to prevent back-out into the joint space.

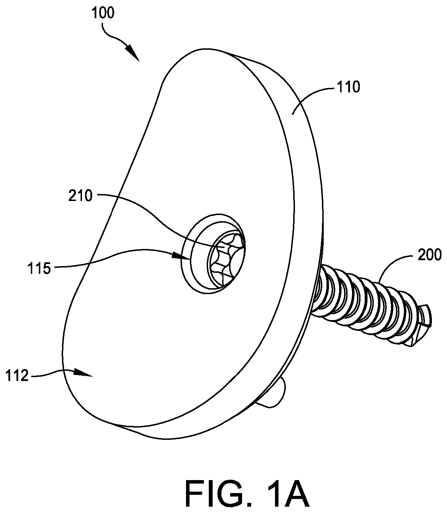

Referring to FIGS. 1A-1D, an improved glenoid implant **100** for implantation in a glenoid according to the present disclosure is provided. The implant **100** comprises an articular body **110,** a metal tray **120;** and one or more holes **115** provided in the articular body **110.** The articular body **110** comprises an articulation surface **112** and a bone-facing surface **113** on the opposite side of the articular body **110.** The metal tray **120** is attached to the bone-facing surface **113.** The one or more holes **115** extend from the articulation surface **112** through the articular body **110** and the metal tray **120.** A screw **200** can be placed through the hole **115** to secure the implant **100** to a glenoid. Although an embodiment with one hole **115** is illustrated, the articular body **110** can have more than one hole **115** to accommodate more than one screw.

In each of the one or more holes **115,** a portion **125** of the metal tray **120** that forms a part of the hole **115** (i.e., the portion of the hole **115** that is extending through the metal tray **120**) is threaded for engaging a threaded screw head **210.** The threaded screw head **210** can be a locking type and the threads on the screw head and the threads on the metal tray portion of the one or more holes **115** are preferably configured to allow polyaxial locking of the screw. Allowing the screw to be locked at various angles allows the screw to be driven in the best trajectory relative to the patient anatomy. An example of such thread design is Wright Medical Technology's Ortholoc^{™} design, which allows screw angulation of +/-15 degrees in any direction.

In other embodiments, the locking of the screw can be monoaxial.

There are also other ways of locking a screw head into the articular body **115** that can be implemented in some embodiments. An example is a snap ring that can be positioned inside the hole **115** that the screw snaps into. The snap ring option can be useful in the all-poly articular body in the glenoid implant **100A** discussed below.

In some embodiments, the internal walls of the one or more holes **115** are not threaded at all. In such embodiments, the diameter of the holes can be sized so that the threaded head of the screws can self-tap into the holes **115.**

In some embodiments, the articular body **110** is formed of a high-modulus polymer. Some examples of such polymer are polyether ether ketone (PEEK), high-modulus polyethylene (HMPE), and ultra-high-molecular-weight polyethylene (UHMWPE), etc. that are selected to provide the desired performance for the articulation surface **112.** All references to UHMWPE herein includes all variants of UHMWPE in orthopedic application such as vitamin E diffused UHMWPE.

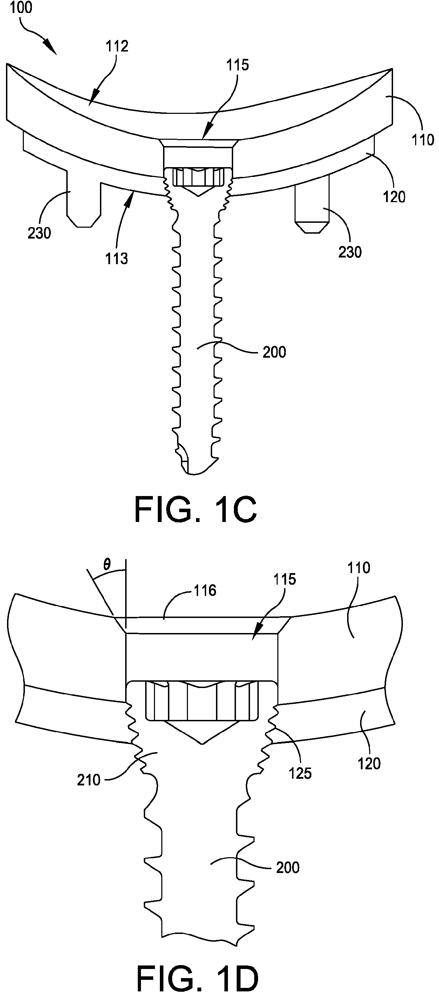

Referring to FIG. 1D, which is an enlarged sectional illustration of the hole **115** and a bone screw **200** positioned therein, in some embodiments, each of the one or more holes **115** in the articular body has an opening at the articulation surface, the opening defining a rim **116,** wherein the rim is preferably chamfered to minimize the friction between the articulation surface **112** and the opposing humeral head. In some embodiments, the chamfer angle ***θ*** can be about 45 degrees. The width of the chamfer is no greater than 1 mm to maximize the articulation surface area. In some embodiments, the chamfer is preferably between about 0.3 - 0.2 mm.

The diameter of the hole **115** should not be too large as that would diminish the bearing function of the articulation surface **112.** The diameter of the hole **115** also cannot be too small as it needs to accommodate a screw of sufficient diameter to establish a desired primary fixation of the implant **100.** The diameter of the hole **115** can be optimized based on these parameters. For example, in many patients, screws of 3.5-4.0 mm minimum diameter would be desired and a hole with a minimum diameter of 5 mm would accommodate such screws.

In some embodiments, the metal tray **120** can be made from titanium, stainless steel, an alloy of titanium, and/or an alloy of cobalt-chromium. In some embodiments, the metal tray **120** comprises exposed surfaces, such as the bone-facing surface **113,** that are coated with a coating that promotes bone in-growth. Some examples of such porous metallic material are Tritanium^{®} by Stryker Corporation and ADAPTIS^{™} by Wright Medical Technology.

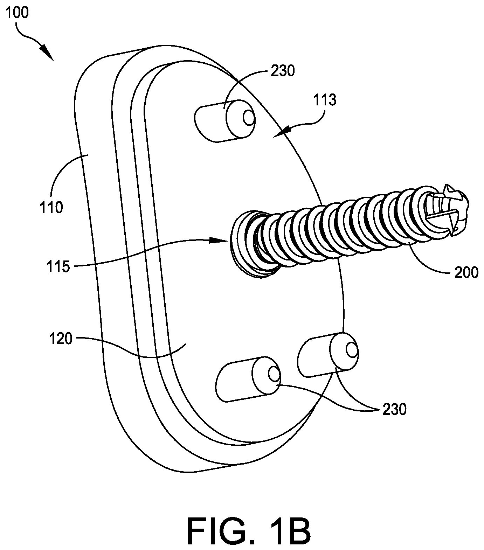

Referring to FIGS. 1B and 1C, in some embodiments, the metal tray **120** further comprises one or more peripheral fixation features **230** extending therefrom in a direction that is away from the articular body **110.** The one or more peripheral fixation features help provide further enhanced fixation of the implant **100** by preventing or minimizing rocking or micro-motion at the interface between the glenoid implant **100** and the glenoid.

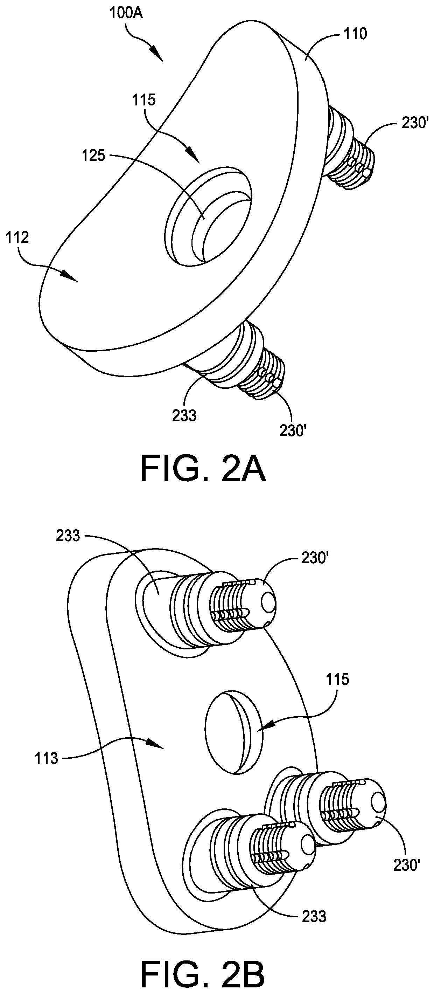

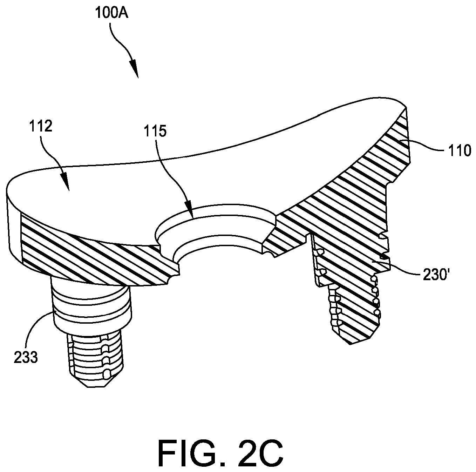

In some embodiments of the glenoid implant **100,** the one or more peripheral fixation features **230** are posts or pegs. In some embodiments, the one or more peripheral fixation features can include a plurality of cement pockets. The illustrated peripheral fixation features **230** do not show such cement pockets but they can look something like the cement pockets **233** on the peripheral fixation features **230'** of the glenoid implant **100A** discussed below. (See FIGS. 2A-2C).

In some embodiments, the one or more peripheral fixation features **230** are integrally formed with the metal tray **120** so that the peripheral fixation features **230** are also formed of metal. In some embodiments, the one or more peripheral fixation features can be protruding extensions of the polymer articular body **110** and the metal tray **120** are configured with through holes that allow the peripheral fixation features to extend through the metal tray **120.**

In some embodiments where the one or more peripheral fixation features are integrally formed with the metal tray **120,** the one or more peripheral fixation features can be an annular ring-like structure that extends from the bone-facing surface **113** of the metal tray **120.** The annular ring-like structure can be configured as a continuous ring structure or can be configured into two or more segments. In some embodiments, the annular ring-like structure can be provided in combination with posts **230.** An example of such structure is seen in the glenoid implant **100B** embodiment discussed below.

In some embodiments where the one or more peripheral fixation features **230, 235** are integrally formed with the metal tray **120,** some portion of or all of the exposed metal surfaces can be coated with a porous metal coating, such as those mentioned above, that can promote bone in-growth.

Referring to FIGS. 2A-2C, an improved glenoid implant **100A** for implantation in a glenoid according to another example not forming part of the present invention is provided. The implant **100A** comprises an articular body **110** with one or more holes **115** provided in the articular body **110.** The articular body **110** is formed of a high-modulus polymer. A screw **200** can be placed through the hole **115** to secure the implant **100A** to a glenoid. The articular body **110** comprises an articulation surface **112** and a bone-facing surface **113** on the opposite side of the articular body **110.** The one or more holes **115** extend from the articulation surface **112** through the articular body **110** to the bone-facing surface **113.** Unlike the glenoid implant **100,** the glenoid implant **100A** does not include a metal tray. The hole **115** can be threaded for receiving a threaded head of a locking screw as discussed above in reference to the glenoid implant **100.** As the glenoid implant **100A** does not have a metal tray and is an all-polymer implant, the one or more holes **115** can be configured without any screw threads and sized so that the threaded head of the locking screw can self-tap into the hole **115.**

In some embodiments, the glenoid implant **100A** can include one or more peripheral fixation features **230'** that are posts or pegs. In some embodiments, the one or more peripheral fixation features **230'** can include a plurality of cement pockets **233.**

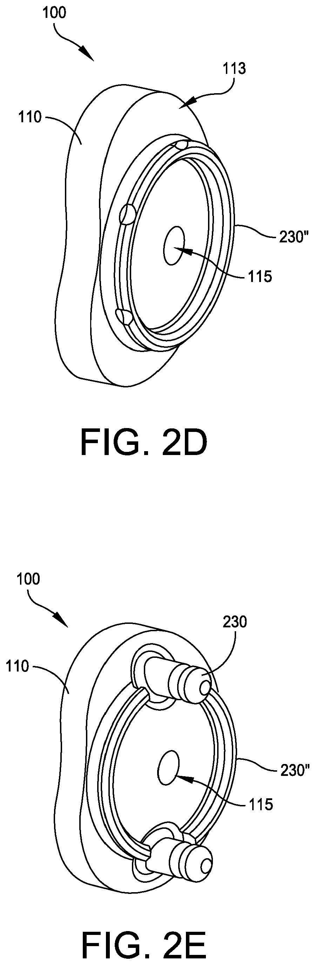

In some embodiments of the glenoid implants **100A,** the one or more peripheral fixation features **230'** can be provided in an annular ring-like structure that extends from the bone-facing surface **113** of the glenoid implant **100A.** The annular ring-like structure can be configured as a continuous ring structure **230"** as shown in the example in FIG. 2D. In some embodiments, the annular ring-like structure can be configured into two or more segments. In an exemplary illustration shown in FIG. 2E, the annular ring-like structure **230‴** is configured into two segments in combination with posts **230.** An example of such structure is seen in the glenoid implant **100B** embodiment discussed below.

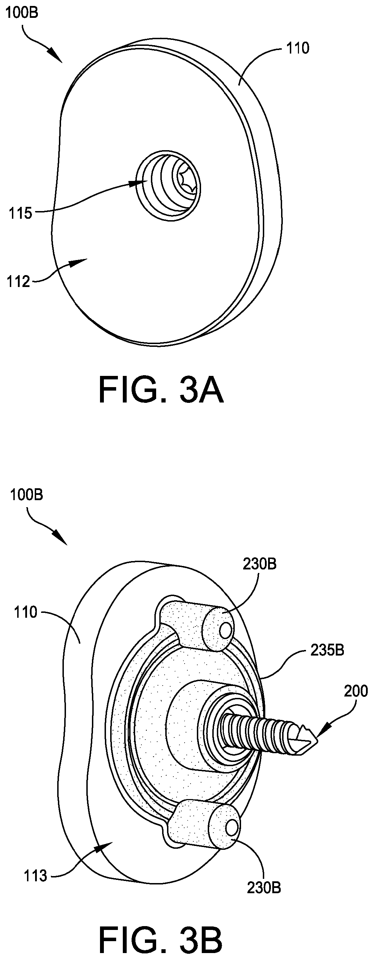

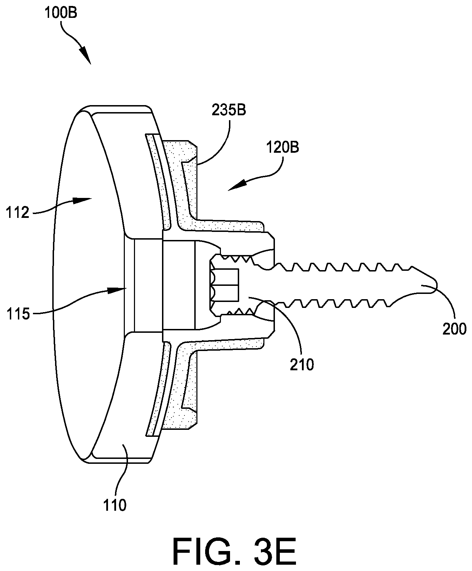

Referring to FIGS. 3A-3E, an improved glenoid implant **100B** for implantation in a glenoid according to another embodiment is provided. The implant **100B** comprises an articular body **110,** a metal tray **120B;** and one or more holes **115** provided in the articular body **110**. The articular body **110** comprises an articulation surface **112** and a bone-facing surface **113** on the opposite side of the articular body **110.** The metal tray **120B** is attached to the bone-facing surface **113.** The one or more holes **115** extend from the articulation surface **112** through the articular body **110** and the metal tray **120B.** A screw **200** can be placed through the hole **115** to secure the implant **100B** to a glenoid. Although an embodiment with one hole **115** is illustrated, the articular body **110** can have more than one hole **115** to accommodate more than one screw.

In each of the one or more holes **115,** a portion **125** of the metal tray **120B** that forms a part of the hole **115** (i.e., the portion of the hole **115** that is extending through the metal tray **120**) is threaded for engaging a threaded screw head **210.**

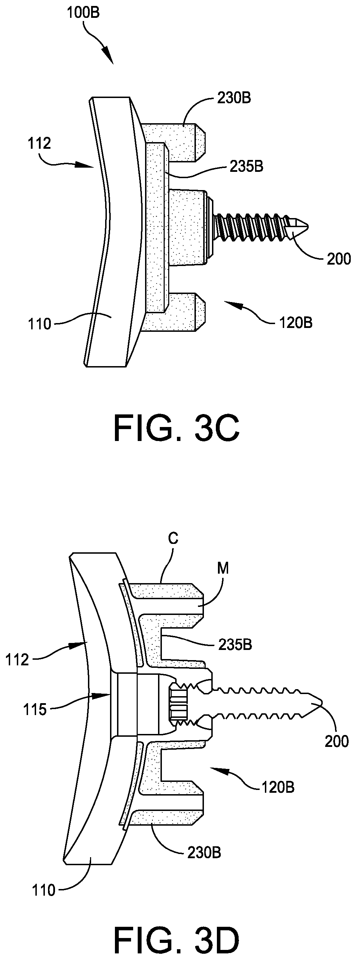

Referring to FIGS. 3C and 3D, the metal tray **120B** in this embodiment includes a combination of an annular ring-like structure **235B** and posts **230B** as the peripheral fixation features for the implant **100B.** This configuration allows the implant **100B** to be a semi-inlay hybrid glenoid implant.

The polymer articular body **110** mates with the glenoid in an onlay arrangement while the metal tray **120B** which protrudes from the articular body **110** toward the glenoid mates with the glenoid in an inlay arrangement. The surface of the metal tray **120B** including the annular ring-like structure **235B** and the posts **230B** are coated with a porous metallic coating such as Tritanium^{®} by Stryker Corporation and ADAPTIS^{™} by Wright Medical Technology that promotes bone in-growth. In the sectional view shown in FIG. 3D, the porous coating portion **C** and the core metal portion **M** of the metal tray **120B** are illustrated.

As in the other embodiments described above, the screw **200** can be a locking type with a threaded screw head **210** that can lock at various angles.

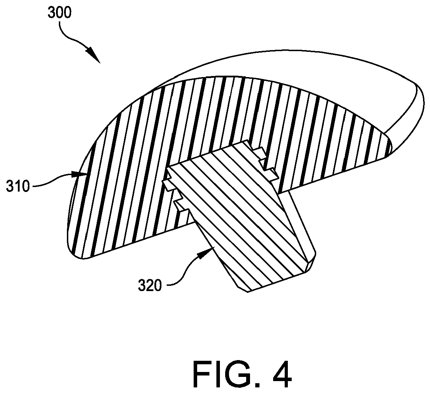

The various embodiments of a glenoid implant disclosed herein can be part of an implant system for shoulder joint repair. Such implant system can comprise a first articular component that is a glenoid implant disclosed herein and a second articular component that is a humeral head implant. FIG. 4 is a sectional view of a such humeral head implant **300,** configured to engage the articular surface **112** of the glenoid bearing implants **100, 100A** of the present disclosure. In some embodiments, at least the articulating surface **310** of the humeral head implant **300** is virgin PEEK to be paired with the glenoid bearing implant **100, 100A,** where at least the articulating surface **112** of the glenoid bearing implant **100, 100A** is formed by UHMWPE. In some embodiments, highly cross-linked UHMWPE-X3 manufactured by Stryker Corporation is preferred for the articulating surface **112.** This material combination should yield a durable implant system with prolonged life. In 3 million cycles (Mc) of wear testing in the shoulder, this material combination (PEEK / UHMWPE-X3) resulted in 85% less wear than the standard CoCr / UHMWPE-X3 combination. The articulating surface **310** can be either a monolithic PEEK or an overmold of a titanium taper component **320.** Either configuration would eliminate the current concern of dissimilar materials on either side of taper connection.

The concept of a hole in the polymer bearing is counter-intuitive in orthopedics since usually the combination of a metal head and polymer bearing creates sufficiently high contact stresses that a hole in the bearing would lead to increased wear. The use of a polymer head may make this design feasible. It is also likely only to be feasible in the shoulder or other joints which do not experience loads that are multiples of body weight.

The preferred embodiment of the PEEK head is a titanium alloy taper that is overmolded with PEEK. The titanium taper component has ridges which allow interdigitation of the PEEK into the titanium.

### Reverse Shoulder Arthroplasty (RSA) Applications

In RSA applications, the bearing implant with one or more screw hole in the articulation surface can be secured to the humeral stem implant. The screw fixation for the bearing implant would provide enhanced stable fixation of the bearing implant in RSA application also.

Although the devices, kits, systems, and methods have been described in terms of exemplary embodiments, they are not limited thereto.

## Claims

1. A glenoid implant (100) comprising:
an articular body (110) comprising an articulation surface (112) and a bone-facing surface (113) on opposite side of the articular body;
a metal tray (120) attached to the bone-facing surface; **characterized by** one or more holes (115) provided in the articular body extending from the articulation surface through the articular body and the metal tray to accommodate a screw (200) therethrough,
wherein each of the one or more holes has an opening at the articulation surface and the opening defines a rim (116) that is chamfered to minimize friction between the articulation surface and an opposing humeral head.

2. The glenoid implant of Claim 1, wherein in each of the one or more holes, a portion that is extending through the metal tray is threaded for engaging a threaded screw head (210).

3. The glenoid implant of Claim 2, wherein in each of the one or more holes, a portion that is extending through the metal tray is threaded to engage with a polyaxial locking screw head.

4. The glenoid implant of Claim 1, wherein the articular body is formed of a high-modulus polymer.

5. The glenoid implant of Claim 1, wherein the articular body is formed of high-modulus polyethylene (HMPE) or ultra-high-molecular-weight polyethylene (UHMWPE).

6. The glenoid implant of Claim 1, wherein the metal tray comprises exposed surfaces that are coated with a porous metal coating that can promote bone in-growth.

7. The glenoid implant of Claim 1, wherein the metal tray further comprises one or more peripheral fixation features (230) extending therefrom in a direction that is away from the articular body.

8. The glenoid implant of Claim 7, wherein the one or more peripheral fixation features are posts.

9. The glenoid implant of Claim 8, wherein the one or more peripheral fixation features include a plurality of cement pockets (233).

10. The glenoid implant of Claim 7, wherein the one or more peripheral fixation features is an annular ring.

11. The glenoid implant of Claim 10, wherein the annular ring is configured into two or more segments.

12. The glenoid implant of Claim 7, wherein the one or more peripheral fixation features are coated with a porous metal coating that can promote bone in-growth.

13. The glenoid implant of Claim 1, wherein the rim is chamfered with a chamfer angle of 45 degrees.

14. The glenoid implant of Claim 1, wherein the rim is chamfered with a chamfer width not greater than 1 mm.

15. The glenoid implant of Claim 14, wherein the chamfer width is between 0.3 - 0.2 mm.

## Patentansprüche

1. Glenoidimplantat (100), umfassend:
einen Gelenkkörper (110), der eine Gelenkoberfläche (112) und eine dem Knochen zugewandte Oberfläche (113) auf der gegenüberliegenden Seite des Gelenkkörpers umfasst;
eine Metallschale (120), die an der dem Knochen zugewandten Oberfläche angebracht ist; **gekennzeichnet durch**
ein oder mehrere Löcher (115), die in dem Gelenkkörper bereitgestellt sind und sich von der Gelenkoberfläche durch den Gelenkkörper und die Metallschale erstrecken, um eine Schraube (200) da hindurch aufzunehmen,
wobei jedes des einen oder der mehreren Löcher eine Öffnung an der Gelenkoberfläche aufweist und die Öffnung einen Rand (116) definiert, der abgeschrägt ist, um die Reibung zwischen der Gelenkoberfläche und einem gegenüberliegenden Humeruskopf zu minimieren.

2. Glenoidimplantat nach Anspruch 1, wobei in jedem des einen oder der mehreren Löcher ein Abschnitt, der sich durch die Metallschale erstreckt, mit Gewinde zum Eingriff mit einem mit Gewinde versehenen Schraubenkopf (210) versehen ist.

3. Glenoidimplantat nach Anspruch 2, wobei in jedem des einen oder der mehreren Löcher ein Abschnitt, der sich durch die Metallschale erstreckt, mit Gewinde zum Eingriff mit einem polyaxialen Feststellschraubenkopf versehen ist.

4. Glenoidimplantat nach Anspruch 1, wobei der Gelenkkörper aus einem hochmoduligen Polymer gebildet ist.

5. Glenoidimplantat nach Anspruch 1, wobei der Gelenkkörper aus hochmoduligem Polyethylen (HMPE) oder ultrahochmolekularem Polyethylen (UHMWPE) gebildet ist.

6. Glenoidimplantat nach Anspruch 1, wobei die Metallschale freiliegende Oberflächen umfasst, die mit einer porösen Metallbeschichtung beschichtet sind, die Knocheneinwuchs fördern kann.

7. Glenoidimplantat nach Anspruch 1, wobei die Metallschale ferner ein oder mehrere periphere Fixierungsmerkmale (230) umfasst, die sich von dieser in eine Richtung weg von dem Gelenkkörper erstrecken.

8. Glenoidimplantat nach Anspruch 7, wobei das eine oder die mehreren peripheren Fixierungsmerkmale Pfosten sind.

9. Glenoidimplantat nach Anspruch 8, wobei das eine oder die mehreren peripheren Fixierungsmerkmale eine Vielzahl von Zementtaschen (233) aufweisen.

10. Glenoidimplantat nach Anspruch 7, wobei das eine oder die mehreren peripheren Fixierungsmerkmale ein kreisförmiger Ring ist.

11. Glenoidimplantat nach Anspruch 10, wobei der kreisförmige Ring in zwei oder mehr Segmente konfiguriert ist.

12. Glenoidimplantat nach Anspruch 7, wobei das eine oder die mehreren peripheren Fixierungsmerkmale mit einer porösen Metallbeschichtung beschichtet sind, die Knocheneinwuchs fördern kann.

13. Glenoidimplantat nach Anspruch 1, wobei der Rand mit einem Fasenwinkel von 45 Grad abgeschrägt ist.

14. Glenoidimplantat nach Anspruch 1, wobei der Rand mit einer Fasenbreite von nicht mehr als 1 mm abgeschrägt ist.

15. Glenoidimplantat nach Anspruch 14, wobei die Fasenbreite zwischen 0,3 und 0,2 mm liegt.

## Revendications

1. Implant glénoïdien (100) comprenant :
un corps articulaire (110) comprenant une surface d'articulation (112) et une surface faisant face à un os (113) sur un côté opposé du corps articulaire ;
un plateau métallique (120) fixé contre la surface faisant face à un os ; **caractérisé par** un ou plusieurs orifices (115) prévus dans le corps articulaire s'étendant depuis la surface d'articulation à travers le corps articulaire et le plateau métallique pour loger une vis (200) à travers celui-ci,
dans lequel chacun des un ou plusieurs orifices présente une ouverture au niveau de la surface d'articulation et l'ouverture définit un rebord (116) qui est chanfreiné pour minimiser une friction entre la surface d'articulation et une tête humérale opposée.

2. Implant glénoïdien selon la revendication 1, dans lequel dans chacun des un ou plusieurs orifices, une partie qui s'étend à travers le plateau métallique est filetée pour engager une tête de vis filetée (210).

3. Implant glénoïdien selon la revendication 2, dans lequel dans chacun des un ou plusieurs orifices, une partie qui s'étend à travers le plateau métallique est filetée pour s'engager avec une tête de vis de blocage poly-axiale.

4. Implant glénoïdien selon la revendication 1, dans lequel le corps articulaire est formé dans un polymère à module élevé.

5. Implant glénoïdien selon la revendication 1, dans lequel le corps articulaire est formé dans un polyéthylène à module élevé (HMPE, *high-modulus polyethylene*) ou un polyéthylène à poids moléculaire ultra élevé (UHMWPE, *ultra-high-molecular-weight polyethylene*)*.*

6. Implant glénoïdien selon la revendication 1, dans lequel le plateau métallique comprend des surfaces exposées qui sont revêtues d'un revêtement métallique poreux qui peut favoriser une croissance intérieure osseuse.

7. Implant glénoïdien selon la revendication 1, dans lequel le plateau métallique comprend en outre un ou plusieurs moyens de fixation périphériques (230) s'étendant depuis celui-ci dans une direction qui est éloignée du corps articulaire.

8. Implant glénoïdien selon la revendication 7, dans lequel les un ou plusieurs moyens de fixation périphériques sont des plots.

9. Implant glénoïdien selon la revendication 8, dans lequel les un ou plusieurs moyens de fixation périphériques incluent une pluralité de poches de ciment (233).

10. Implant glénoïdien selon la revendication 7, dans lequel les un ou plusieurs moyens de fixation périphériques est une bague annulaire.

11. Implant glénoïdien selon la revendication 10, dans lequel la bague annulaire est configurée en deux ou plusieurs segments.

12. Implant glénoïdien selon la revendication 7, dans lequel les un ou plusieurs moyens de fixation périphériques sont revêtus d'un revêtement métallique poreux qui peut favoriser une croissance intérieure osseuse.

13. Implant glénoïdien selon la revendication 1, dans lequel le rebord est chanfreiné avec un angle de chanfrein de 45 degrés.

14. Implant glénoïdien selon la revendication 1, dans lequel le rebord est chanfreiné avec une largeur de chanfrein qui n'est pas supérieure à 1 mm.

15. Implant glénoïdien selon la revendication 14, dans lequel la largeur de chanfrein se situe entre 0,3 et 0,2 mm.
